# EUROPÄISCHE PATENTSCHRIFT

(11) **EP 1 165 469 B1**
(45) Veröffentlichungstag und Bekanntmachung des Hinweises auf die Patenterteilung: **13.08.2003**
(21) Anmeldenummer: 00925169.5
(22) Anmeldetag: 05.04.2000
(51) Int. Cl.: C07C 2/10

(54) **VERFAHREN ZUR OLIGOMERISIERUNG VON C2- BIS C8-OLEFINEN**
METHOD FOR OLIGOMERISING VON C2- TO C8-OLEFINS
PROCEDE D'OLIGOMERISATION D'OLEFINES C2 A C8

(30) Priorität: 06.04.1999 DE 19915357
(43) Veröffentlichungstag der Anmeldung: 02.01.2002
(73) Patentinhaber: BASF AKTIENGESELLSCHAFT, 67056 Ludwigshafen (DE)
(72) Erfinder: KEUSER, Ulrich, D-67273 Weisenheim (DE); BROX, Wolfgang, D-69118 Heidelberg (DE); NEUMANN, Hans-Peter, D-67067 Ludwigshafen (DE); SCHULZ, Ralf, D-67346 Speyer (DE); WALTER, Marc, D-67227 Frankenthal (DE)
(74) Vertreter: Kinzebach, Werner, Dr.
(86) Internationale Anmeldenummer: EP0003023
(87) Internationale Veröffentlichungsnummer: WO00059849

(56) Entgegenhaltungen:
- US-A- 4 533 651
- US-A- 4 628 138
- US-A- 5 177 282
- US-A- 5 849 972

## Beschreibung

Die vorliegende Erfindung betrifft ein Verfahren zur Oligomerisierung von C₂- bis C₈-Olefinen an einem Nickel enthaltenden heterogenen Katalysator.

Olefine mit 2 bis 8 Kohlenstoffatomen oder deren Gemische stehen in großen Mengen sowohl aus FCC-Anlagen (Fluidized Catalyst Cracking) als auch aus Steamcrackern zur Verfügung. Es ist bekannt, die C₄-Fraktion, d.h. ein im Wesentlichen aus Butenen und Butanen bestehendes Gemisch, gegebenenfalls nach Abtrennung des iso-Butens, zur Herstellung von Oligomeren, insbesondere von Octenen und Dodecenen, zu verwenden. Sowohl die Octene als auch die Dodecene können nach der Hydroformylierung und anschließender Hydrierung zu den entsprechenden Alkoholen z.B. zur Herstellung von Weichmachern oder Tensiden verwendet werden.

Die Oligomerisierung wird großtechnisch entweder unter homogener oder heterogener Katalyse durchgeführt. Das homogen katalysierte Verfahren weist den Nachteil auf, dass der Katalysator vom Reaktionsgemisch getrennt werden muß. Dieser Abtrennungsschritt verursacht Abfallströme, die aufwendig aufgearbeitet werden müssen. Außerdem läßt sich der homogene Katalysator nicht regenerieren.

Die beschriebenen Nachteile bestehen bei der heterogen katalysierten Olefinoligomerisierung nicht. Die wichtigsten industriell ausgeübten heterogen katalysierten Olefinoligomerisierungsverfahren sind z.B. in A. Chauvel und G. Lefebvre, Petrochemical Process, Edition Technip (1989), S. 183-187 und F. Asinger, Die petrolchemische Industrie, Akademie-Verlag (1971), S. 278-299 angegeben.

Aus der DE-4339713 ist ein Verfahren zum Oligomerisieren von unverzweigten C₂- bis C₈-Olefinen an einem Festbettkatalysator bei erhöhtem Druck und erhöhter Temperatur bekannt, wobei der Katalysator als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumoxid enthält. Nach diesem Verfahren kann die Oligomerisierung von Butenen mit sehr guter Selektivität bezüglich linearer Produkte durchgeführt werden. Die DE-4339713 empfiehlt, den frisch hergestellten Katalysator vor dem Einsatz einer Konditionierung im trockenen Stickstoffstrom, z.B. bei Atmosphärendruck und Temperaturen von 200 bis 500°C zu unterwerfen, um noch enthaltenes Wasser aus dem Katalysator zu entfernen. Es hat sich gezeigt, dass insbesondere bei adiabatischer Führung des Verfahrens nach der DE-4339713 die erzielbare Standzeit des Katalysators verbesserungswürdig ist.

Der vorliegenden Erfindung liegt daher die Aufgabe zugrunde, ein Verfahren zur Oligomerisierung von C₂- bis C₈-Olefinen an einem Nickel enthaltenden heterogenen Katalysator vorzuschlagen, das eine möglichst lange Katalysatorstandzeit aufweist.

Es wurde nun überraschend gefunden, dass die Katalysatorstandzeit erheblich verlängert wird, wenn der frisch hergestellte bzw. konditionierte Katalysator vor dem Inkontaktbringen mit dem eigentlichen Einsatzgemisch einer Vorbehandlung unterzogen wird, die das Inkontaktbringen des Katalysators mit einem gegenüber dem Einsatzgemisch olef inärmeren Kohlenwasserstoffgemisch umfasst.

Demzufolge ist Gegenstand der Erfindung ein Verfahren zur Oligomerisierung von C₂- bis C₈-Olefinen an einem Nickel enthaltenden heterogenen Katalysator, das eine Katalysatorvorbehandlungsphase, bei der der Katalysator mit einem ersten Kohlenwasserstoffgemisch, das einen Olefingehalt von weniger als 50 Gew.-% und einen Gehalt an Diolefinen und Alkinen von weniger als 0,1 Gew.-% aufweist (im Folgenden: "olefinarmes Kohlenwasserstoffgemisch"), in Kontakt gebracht wird, und eine, vorzugsweise im Wesentlichen stationäre, Betriebsphase umfasst, bei der ein Strom eines zweiten Kohlenwasserstoffgemisches, das einen Gehalt an C₂- bis C₈-Olefinen von 50 bis 100 Gew.-% und einen Gehalt an Diolefinen und Alkinen von weniger als 10 Gew.-ppm aufweist (im Folgenden: "olefinreiches Kohlenwasserstoffgemisch"), mit dem Katalysator in Kontakt gebracht wird. Es ist bevorzugt, das olefinarme Kohlenwasserstoffgemisch vom Strom des olefinreichen Kohlenwasserstoffgemisches verdrängen zu lassen. Die Katalysatorvorbehandlungsphase ist dabei gegenüber der Betriebsphase zeitlich kurz, z.B. 12 bis 72 Stunden Katalysatorvorbehandlungsphase gegenüber 1 bis 2 Jahren Betriebsphase. In der Regel ist bevorzugt, dass das olefinarme und das olefinreiche Kohlenwasserstoffgemisch im Wesentlichen aus Kohlenwasserstoffen der gleichen Anzahl von C-Atomen bestehen. Insbesondere handelt es sich bei dem olefinarmen und dem olefinreichen Kohlenwasserstoffgemisch im Wesentlichen um Gemische von C₄-Kohlenwasserstoffen.

Es wird vorliegend der Begriff "Kohlenwasserstoffgemisch" verwendet, weil es sich bei den großtechnisch zugänglichen, zur Durchführung des erfindungsgemäßen Verfahrens geeigneten Kohlenwasserstoffströmen in der Regel um Gemische handelt. Es versteht sich, dass das erfindungsgemäße Verfahren auch mit reinen Kohlenwasserstoffen durchgeführt werden kann, die den angegebenen Bedingungen genügen.

Das erfindungsgemäße verfahren kann zwischen der Katalysatorvorbehandlungsphase und der Betriebsphase eine Intermediärphase umfassen, bei der der Katalysator mit einem Kohlenwasserstoffgemisch zeitlich veränderlicher Zusammensetzung in Kontakt gebracht wird, die sich kontinuierlich oder stufenweise von der eines olefinarmen Kohlenwasserstoffgemisches zu der eines olefinreichen Kohlenwasserstoffgemisches verändert. In der Betriebsphase wird vorzugsweise ein im wesentlichen stationärer Betriebszustand aufrechterhalten. "Im Wesentlichen stationär" bedeutet, dass - von natürlichen Fluktuationen abgesehen - die Zusammensetzung der Edukt- und Produktströme möglichst konstant gehalten wird.

Die Katalysatorvorbehandlungsphase und die Betriebsphase können räumlich und/oder zeitlich getrennt voneinander durchgeführt werden. Dieser Fall liegt beispielsweise regelmäßig vor, wenn die Katalysatorvorbehandlungsphase im zeitlichen und/oder räumlichen Zusammenhang mit der Katalysatorherstellung erfolgt. Es ist in diesem Fall bevorzugt, dass der Katalysator beim Transport und/oder während der Lagerung mit olefinarmem Kohlenwasserstoffgemisch bedeckt gehalten wird. Im Allgemeinen ist es jedoch bevorzugt, dass die Katalysatorvorbehandlungsphase und die Betriebsphase im gleichen Reaktor durchgeführt werden. Es ist weiter bevorzugt, die Katalysatorvorbehandlungsphase, vorzugsweise einschließlich einer nachstehend näher erläuterten Konditionierung des Katalysators, unmittelbar vor Beginn der Betriebsphase durchzuführen.

Die verwendbaren heterogenen Nickel enthaltenden Katalysatoren können unterschiedliche Struktur aufweisen. Es kommen an sich bekannte Katalysatoren in Betracht, wie sie in C.T. O'Connor et al., Catalysis Today, Bd.6 (1990), S.336-338 beschrieben sind. Insbesondere werden trägergebundene Nickelkatalysatoren eingesetzt. Die Trägermaterialien können z.B. Kieselsäure, Tonerde, Aluminosilicate, Aluminosilicate mit Schichtstrukturen und Zeolithe, wie Mordenit, Faujasit, zeolith X, Zeolith-Y und ZSM-5, Zirkoniumoxid, das mit Säuren behandelt ist, oder sulfatiertes Titandioxid sein. Besonders geeignet sind Fällungskatalysatoren, die durch Mischen wässriger Lösungen von Nickelsalzen und Silicaten, z.B. Natriumsilicat mit Nickelnitrat, und gegebenenfalls Aluminiumsalzen, wie Aluminiumnitrat, und Calcinieren erhältlich sind. Weiterhin sind Katalysatoren verwendbar, die durch Einlagerung von Ni²⁺-Ionen durch Ionenaustausch in natürliche oder synthetische Schichtsilicate, wie Montmorillonite, erhalten werden. Geeignete Katalysatoren können auch durch Imprägnieren von Kieselsäure, Tonerde oder Alumosilicaten mit wässrigen Lösungen löslicher Nickelsalze, wie Nickelnitrat, Nickelsulfat oder Nickelchlorid, und anschließende Calcinierung erhalten werden.

Nickeloxid enthaltende Katalysatoren sind bevorzugt. Besonders bevorzugt sind Katalysatoren, die im Wesentlichen aus NiO, SiO₂, TiO₂ und/oder ZrO₂ sowie gegebenenfalls Al₂O₃ bestehen. Derartige Katalysatoren sind insbesondere bevorzugt, wenn das erfindungsgemäße Verfahren zur Oligomerisierung von Butenen herangezogen wird. Sie führen zu einer Bevorzugung der Dimerisierung gegenüber der Bildung höherer Oligomere und liefern überwiegend lineare Produkte. Am meisten bevorzugt ist ein Katalysator, der als wesentliche aktive Bestandteile 10 bis 70 Gew.-% Nickeloxid, 5 bis 30 Gew.-% Titandioxid und/oder Zirkondioxid, 0 bis 20 Gew.-% Aluminiumoxid und als Rest Siliciumdioxid enthält. Ein solcher Katalysator ist durch Fällung der Katalysatormasse bei pH 5 bis 9 durch Zugabe einer Nickelnitrat enthaltenden wässrigen Lösung zu einer Alkaliwasserglaslösung, die Titandioxid und/oder Zirkondioxid enthält, Filtrieren, Trocknen und Tempern bei 350 bis 650°C erhältlich. Zur Herstellung dieser Katalysatoren wird im einzelnen auf die DE-4339713 verwiesen. Auf die Offenbarung dieser Druckschrift und den darin zitierten Stand der Technik wird vollinhaltlich Bezug genommen.

Der Katalysator liegt vorzugsweise in stückiger Form, z.B. in Form von Tabletten, z.B. mit einem Durchmesser von 2 bis 6 mm und einer Höhe von 3 bis 5 mm, Ringen mit z.B. 5 bis 7 mm Außendurchmesser, 2 bis 5 mm Höhe und 2 bis 3 mm Lochdurchmesser, oder Strängen unterschiedlicher Länge eines Durchmessers von z.B. 1,5 bis 5 mm, vor. Derartige Formen werden auf an sich bekannte Weise durch Tablettierung oder Extrusion, meist unter Verwendung eines Tablettierhilfsmittels, wie Graphit oder Stearinsäure, erhalten.

Das erfindungsgemäße Verfahren geht von einem frisch hergestellten, regenerierten oder konditionierten Katalysator aus. Die Herstellung geeigneter Katalysatoren ist vorstehend beschrieben bzw. ist an sich bekannt. Unmittelbar nach der Herstellung befindet sich der Katalysator in einem Zustand hoher Aktivität, insbesondere wenn die Herstellung mit einem Wärmebehandlungsschritt endet. Die Regeneration eines gebrauchten Katalysators unter Wiedererlangung einer hohen Aktivität kann z.B. durch sogenanntes "Abbrennen" erfolgen, d.h. durch Erwärmen des Katalysators in einem sauerstoffhaltigen Gasgemisch, z.B. einem Gemisch von O₂ und N₂, auf eine Temperatur von z.B. 300-600°C. Das Verhältnis von O₂ und N₂ in dem angegebenen Gemisch von O₂ und N₂ wird mit Vorteil so gewählt, dass die bei der Behandlung des gebrauchten Katalysators auftretende Wärmetönung weniger als 50°C beträgt.

Im Allgemeinen ist es bevorzugt, den Katalysator im Rahmen der Katalysatorvorbehandlungsphase vor dem Inkontaktbringen mit dem olefinarmen Kohlenwasserstoffgemisch zu konditionieren. Hierzu kann der Katalysator in Gegenwart eines Inertgases auf eine Temperatur von mehr als 100°C, vorzugsweise 150 bis 500°C, erwärmt werden. Das Erwärmen kann einige Stunden bis mehrere Tage, z.B. 6 bis 150 Stunden, vorzugsweise 12 bis 72 Stunden, dauern. Vorzugsweise wird eine Katalysatorschüttung vom Inertgas, das unter Normaldruck oder erhöhtem Druck vorliegen kann, durchströmt. Als Inertgas sind Gase geeignet, die am Katalysator bei der Konditionierungstemperatur im Wesentlichen keine chemische Umwandlung erfahren und mit dem Katalysator im Wesentlichen keine chemische oder physikalische Wechselwirkung eingehen. Geeignet sind z.B. Stickstoff, Argon oder Neon. Vorzugsweise lässt man den Katalysator nach dem Erwärmen anschließend im Inertgasstrom abkühlen, vorzugsweise auf eine Temperatur von weniger als 40 °C.

Konditionierung und Inkontaktbringen mit dem olefinarmen Kohlenwasserstoffgemisch erfolgen mit Vorteil im gleichen Reaktor.

Erfindungsgemäß wird der Katalysator einer Vorbehandlung unterzogen, bevor er mit dem eigentlichen Produkteinsatzgemisch in Kontakt gebracht wird. Bei der Vorbehandlung wird der Katalysator mit einem Kohlenwasserstoffgemisch eines Olefingehaltes von 0 bis weniger als 50 Gew.-%, in Kontakt gebracht. Vorzugsweise enthält das olefinarme Kohlenwasserstoffgemisch weniger als 45 Gew.-%, insbesondere weniger als 25 Gew.-%, besonders bevorzugt weniger als 10 Gew.-% Olefine. Im Allgemeinen sind Olefingehalte bis hinunter zu 3 Gew.-% großtechnisch zugänglich und daher bevorzugt. Das olefinarme Kohlenwasserstoffgemisch besteht zu 50 Gew.-% oder mehr, vorzugsweise 75 Gew.-% oder mehr, insbesondere 90 Gew.-% oder mehr, aus Kohlenwasserstoffen, die der Oligomerisierung am heterogenen Katalysator nicht zugänglich sind, wie Alkanen, z.B. Propan, Butan, Pentan, Hexan, Heptan, Octan, Nonan, Decan, Undecan und/oder Dodekan. Insbesondere besteht es im Wesentlichen, z.B. zu mehr als 95 Gew.-% aus C₄-Kohlenwasserstoffen. Das olefinarme Kohlenwasserstoffgemisch enthält weniger als 0,1 Gew.-%, insbesondere weniger als 100 Gew.-ppm und besonders bevorzugt weniger als 50 Gew.-ppm Diolefine und/oder Alkine, und ist vorzugsweise im Wesentlichen dien- und alkinfrei. Die Entfernung von Dienen und Alkinen kann z.B. durch selektive Hydrierung erfolgen, wie nachstehend im Zusammenhang mit dem olefinreichen Gemisch erläutert ist.

Im Allgemeinen liegt der Siedebereich (bei 1 atm) des olefinarmen Kohlenwasserstoffgemischs zwischen -50°C und 250°C, insbesondere zwischen -20°C und 80°C, besonders bevorzugt zwischen -15°C und 10°C.

Das Inkontaktbringen des Katalysators mit dem olefinarmen Kohlenwasserstoffgemisch erfolgt vorzugsweise bei einem Druck von 1 bis 4 bar und kann bei einer Temperatur von 10 bis 100°C, vorzugsweise weniger als 70°C, insbesondere weniger als 40°C durchgeführt werden. Das olefinarme Kohlenwasserstoffgemisch kann zum Inkontaktbringen mit dem Katalysator sowohl in der Gasphase als auch in der flüssigen Phase vorliegen. Es ist bevorzugt, dass die Katalysatorvorbehandlungsphase unter solchen Bedingungen durchgeführt wird, dass das olefinarme Kohlenwasserstoffgemisch in flüssiger Phase vorliegt.

Ein als olefinarmes Kohlenwasserstoffgemisch für die Zwecke der vorliegenden Erfindung geeignetes Kohlenwasserstoffgemisch kann z.B. durch Vollhydrierung eines C₄-Stroms aus einer FCC-Anlage oder einem Steamcracker erhalten werden. Des weiteren können Feldbutane eingesetzt werden.

Die Katalysatorvorbehandlungsphase kann sowohl vor Einbau des Katalysators in den Reaktor als auch nach Einbau des Katalysators in den Reaktor erfolgen. In einer besonders bevorzugten Ausführungsform der Erfindung wird der Katalysator nach Einbau in den Reaktor mit dem olefinarmen Kohlenwasserstoffgemisch in Kontakt gebracht.

Die großtechnisch zugänglichen Kohlenwasserstoffgemische, die als olefinarmes Kohlenwasserstoffgemisch für die Zwecke der vorliegenden Erfindung geeignet sind, enthalten oftmals Verbindungen, die als Katalysatorgifte wirken und den Oligomerisierungskatalysator deaktivieren. Hierzu zählen sauerstoffhaltige Verbindungen, wie Alkohole, Aldehyde, Ketone und Ether, sowie stickstoffhaltige, schwefelhaltige und halogenhaltige Verbindungen. Die Anwesenheit solcher Katalysatorgifte bei der Katalysatorvorbehandlungsphase würde zu einer unerwünschten Verringerung der Katalysatoraktivität führen.

Gemäß einem bevorzugten Aspekt der Erfindung wird das olefinarme Kohlenwasserstoffgemisch daher vor dem Inkontaktbringen mit dem Katalysator zur Entfernung von Katalysatorgiften über ein Adsorptionsmittel geleitet. Als Adsorptionsmittel sind Molekularsiebe, vorzugsweise mit einem Porendurchmesser von größer als 4 Å bis 15 Å, geeignet. Als Molekularsiebe können kristalline, natürliche Aluminiumsilicate, wie z.B. Schichtgittersilicate, wie auch synthetische Molekularsiebe eingesetzt werden. Weiter sind kommerzielle Molekularsiebe, wie z.B. Typen der Fa. Bayer AG, Dow, Union Carbide, Laporte oder Mobil, geeignet. Diese Molekularsiebe können z.B. Zeolithe vom A-, X- und Y-Typ sein. Ferner sind auch synthetische Molekularsiebe geeignet, die neben Silicium und Aluminium als Hauptbestandteile noch andere Atome als Nebenbestandteile aufweisen. Diese können z.B. durch einen Ionenaustausch mit den austauschbaren Kationen in den Zeolithen eingebaut werden. Beispielhaft sei hier der Austausch mit seltenen Erden, wie z.B. Gallium, Indium oder Lanthan, oder mit Nickel, Cobalt, Kupfer, Zink oder Silber, aufgeführt.

Darüber hinaus können auch synthetische Zeolithe, in welchen andere Atome, wie z.B. Bor oder Phosphor, die durch Kopräzipitation in das Gitter mit eingebaut sind, eingesetzt werden.

Weitere geeignete Adsorptionsmittel sind z.B. Aluminiumoxide, Aluminiumphosphate, Siliciumdioxide, Kieselgur, Titandioxide, zirkondioxide, polymere Adsorbentien und Gemische davon. Das Überleiten des olefinarmen Kohlenwasserstoffgemisches über das Adsorptionsmittel erfolgt zweckmäßigerweise in einem Festbett oder einem Wanderbett. Das olefinarme Kohlenwasserstoffgemisch kann beim Überleiten über das Adsorptionsmittel in gasförmiger oder flüssiger Phase vorliegen, liegt aber vorzugsweise in flüssiger Phase vor.

Die Konzentration an sauerstoffhaltigen, stickstoffhaltigen, schwefelhaltigen und halogenhaltigen Verbindungen im olefinarmen Gemisch beträgt - gegebenenfalls nach der vorstehend erörterten Vorreinigung - beträgt vorzugsweise weniger als 1 Gew.-ppm, insbesondere weniger als 0,5 Gew.-ppm.

Vorzugsweise geschieht das Inkontaktbringen des Katalysators mit dem olefinannen Kohlenwasserstoffgemisch dergestalt, dass eine Schüttung des Katalysators mit dem olefinarmen Kohlenwasserstoffgemisch durchströmt wird, vorzugsweise unter adiabatischen Bedingungen. Beim Inkontaktbringen des Katalysators mit dem olefinarmen Kohlenwasserstoffgemisch wird in der Regel Wärme frei. Die entstehende Wärme muß bei adiabatischer Fahrweise vom olefinarmen Kohlenwasserstoffgemisch abgeführt werden. Es ist bevorzugt, das olefinarme Kohlenwasserstoffgemisch mit einer Temperatur von 10°C bis weniger als 60°C, vorzugsweise weniger als 40°C, einzuführen und das Durchströmen solange fortzusetzen, bis die Temperatur in der Katalysatorschüttung weniger als 60°C, insbesondere weniger als 40°C, beträgt.

Das olefinarme Kohlenwasserstoffgemisch kann nach dem Inkontaktbringen mit dem Katalysator, gegebenenfalls nach Kühlung, ganz oder teilweise zurückgeführt und gegebenenfalls mit frischem olefinarmen Kohlenwasserstoffgemisch verschnitten werden. Letztlich wird es aus dem Verfahren ausgeleitet und verworfen. Es kann z.B. verbrannt oder einem Steamcracker zugeleitet werden.

Nach abgeschlossener Katalysatorvorbehandlungsphase wird der Katalysator in der Betriebsphase mit einem Strom eines Kohlenwasserstoffgemisches eines Gehalts an C₂- bis C₈-Olefinen von 50 bis 100 Gew.-%, vorzugsweise 60 bis 100 Gew.-%, in Kontakt gebracht. Im Allgemeinen handelt es sich beim Olefinanteil im Wesentlichen um ein Olefin, wie Propylen oder ein Gemisch von Olefinen gleicher Anzahl von C-Atomen, wie isomere Butene. In der Betriebsphase erfolgt die Oligomerisierung, insbesondere Dimerisierung oder Trimerisierung, der im olefinreichen Kohlenwasserstoffgemisch enthaltenen C₂- bis C₈-olefine. Das olefinreiche Kohlenwasserstoffgemisch kann neben C₂- bis C₈-Olefinen einen der Oligomerisierung nicht zugänglichen Inertanteil enthalten. Dieser Inertanteil kann Z.B. aus gesättigten Kohlenwasserstoffen, wie Alkanen und/oder Cycloalkanen, bestehen. In der Regel weisen die gesättigten Kohlenwasserstoffe die gleiche Anzahl an C-Atomen oder eine um ein C-Atom höhere oder niedrigere Anzahl an C-Atomen auf wie das Olefin.

Ein bevorzugtes olefinreiches Gemisch enthält 50 bis 100 Gew.-%, vorzugsweise 60 bis 90 Gew.-%, Butene und 0 bis 50 Gew.-%, vorzugsweise 10 bis 40 Gew.-%, Butane. Vorzugsweise umfasst die Butenfraktion weniger als 5 Gew.-%, insbesondere weniger als 3 Gew.-% iSo-Buten (bezogen auf die Butenfraktion). Die Butenfraktion weist im Allgemeinen folgende Zusammensetzung auf (jeweils bezogen auf die Butenfraktion):

| | |
|---|---|
| 1-Buten | 1 bis 50 Gew.-%, |
| cis-2-Buten | 1 bis 50 Gew.-%, |
| trans-2-Buten | 1 bis 99 Gew.-%, |
| iso-Buten | 1 bis 5 Gew.-%. |

Als besonders bevorzugter Einsatzstoff wird das sogenannte Raffinat II verwendet, bei dem es sich um einen iso-Buten-abgereicherten C₄-Schnitt aus einer FCC-Anlage oder einem Steamcracker handelt. Raffinat II weist folgende typische Zusammensetzung auf:

| | |
|---|---|
| i-,n-Butan | 26 Gew.-%; |
| i-Buten | 1 Gew.-% |
| 1-Buten | 26 Gew.-%, |
| trans-2-Buten | 31 Gew.-%, |
| cis-2-Buten | 16 Gew.-%. |

Sind Diolefine oder Alkine im olefinreichen Kohlenwasserstoffgemisch vorhanden, so werden diese vor der Oligomerisierung auf weniger als 10 Gew.-ppm aus demselben entfernt. Sie werden bevorzugt durch selektive Hydrierung, z.B. gemäß EP-81041 und DE-1568542 entfernt, besonders bevorzugt durch eine selektive Hydrierung bis auf einen Restgehalt von unter 5 Gew.-ppm, insbesondere 1 Gew.-ppm.

Aus dem olefinreichen Kohlenwasserstoffgemisch werden zweckmäßigerweise außerdem sauerstoffhaltige Verbindungen, wie Alkohole, Aldehyde, Ketone oder Ether, weitgehend entfernt. Hierzu kann das olefinreiche Kohlenwasserstoffgemisch mit Vorteil über ein Adsorptionsmittel, wie z.B. ein Molekularsieb, insbesondere eines mit einem Porendurchmesser von > 4 Å bis 5 Å, geleitet werden. Hierzu wird auf die vorstehenden Ausführungen im Zusammenhang mit dem olefinarmen Kohlenwasserstoffgemisch verwiesen. Die Konzentration an sauerstoffhaltigen, schwefelhaltigen, stickstoffhaltigen und halogenhaltigen Verbindungen im olefinreichen Kohlenwasserstoffgemisch beträgt vorzugsweise weniger als 1 Gew.-ppm, insbesondere weniger als 0,5 Gew.-ppm.

Die Betriebsphase erfolgt vorzugsweise bei Temperaturen von 30 bis 280°C, insbesondere 30 bis 140°C und besonders bevorzugt von 40 bis 130°C. Sie erfolgt vorzugsweise bei einem Druck von 10 bis 300 bar, insbesondere von 15 bis 100 bar und besonders bevorzugt von 20 bis 80 bar. Der Druck wird dabei zweckmäßigerweise so eingestellt, dass bei der gewählten Temperatur das olefinreiche Kohlenwasserstoffgemisch flüssig oder im überkritischen Zustand vorliegt.

Geeignete gegebenenfalls druckfeste Reaktionsapparaturen für das Inkontaktbringen des Kohlenwasserstoffgemisches mit dem heterogenen Katalysator sind dem Fachmann bekannt. Dazu zählen die allgemein üblichen Reaktoren für Gas/Fest-Reaktionen bzw. Flüssig/Fest-Reaktionen. Geeignet sind z.B. Rohrbündelreaktoren oder Schachtöfen. Aufgrund der geringeren Investitionskosten sind Schachtöfen bevorzugt. Das erfindungsgemäße Oligomerisierungsverfahren kann in einem einzelnen Reaktor durchgeführt werden, wobei der Oligomerisierungskatalysator in einem einzigen oder mehreren Festbetten im Reaktor angeordnet sein kann. Alternativ kann zur Durchführung des erfindungsgemäßen Verfahrens eine Reaktorkaskade aus mehreren, vorzugsweise zwei, hintereinandergeschalteten Reaktoren eingesetzt werden, wobei beim Passieren des bzw. der dem letzten Reaktor der Kaskade vorgeschalteten Reaktors bzw. Reaktoren die Oligomerisierung der Olefine im Reaktionsgemisch nur bis zu einem Teilumsatz betrieben wird und der gewünschte Endumsatz erst beim Passieren des Reaktionsgemisches durch den letzten Reaktor der Kaskade erzielt wird. In den einzelnen Reaktoren der Reaktorkaskade kann der Oligomerisierungskatalysator in einem einzigen oder in mehreren Katalysatorbetten angeordnet sein. Weiterhin können in den einzelnen Reaktoren der Reaktorkaskade unterschiedliche Reaktionsbedingungen hinsichtlich Druck und/oder Temperatur im Rahmen der oben genannten Druck- und Temperaturbereiche eingestellt werden. Außerdem ist es möglich, in den einzelnen Reaktoren der Kaskade unterschiedliche Oligomerisierungskatalysatoren einzusetzen, obgleich die Anwendung des gleichen Katalysators in sämtlichen Reaktoren der Kaskade bevorzugt ist. Bei dem bevorzugten Reaktor handelt es sich in der Regel um ein mit dem Katalysator beschicktes, vertikales zylindrisches Rohr, das von dem olefinreichen Kohlenwasserstoffgemisch z.B. von oben nach unten durchströmt wird.

Nach dem Verlassen des Reaktors bzw. des letzten Reaktors einer Kaskade werden aus dem Reaktoraustrag die gebildeten Oligomere von den nicht umgesetzten Olefinen und gesättigten Kohlenwasserstoffen abgetrennt. Die gebildeten Oligomere können in einem nachfolgenden Vakuumfraktionierungsschritt aufgereinigt werden.

In einer bevorzugten Ausführungsform wird der von den gebildeten Oligomeren befreite Reaktoraustrag, der im Wesentlichen aus nicht umgesetzten Olefinen und gesättigten Kohlenwasserstoffen besteht, vollständig oder zum Teil zurückgeführt. Es ist bevorzugt, das Rückführverhältnis so zu wählen, dass die Konzentration an Oligomeren im Reaktionsgemisch an keiner Stelle des Reaktors (bzw. der Reaktorkaskade) 35 Gew.-%, vorzugsweise 20 Gew.-%, bezogen auf das Reaktions-Kohlenwasserstoffgemisch, übersteigt.

Die Betriebsphase kann mit Vorteil adiabatisch durchgeführt werden. Die Oligomerisierungsreaktion verläuft in der Regel exotherm. Das Reaktionsgemisch erfährt daher beim Durchströmen des Katalysatorbettes eine Temperaturerhöhung. Unter adiabatischer Reaktionsführung wird im Unterschied zur isothermen Raktionsführung, bei der die in einer exothermen Reaktion entstehende Wärmemenge durch Kühlung mittels Kühl- oder Thermostatiervorrichtungen abgeführt wird und so die Temperatur im Reaktor konstant, d.h. isotherm, gehalten wird, eine Betriebsweise verstanden, bei der die in einer exothermen Reaktion freiwerdende Wärmemenge von der Reaktionsmischung im Reaktor aufgenommen und keine Kühlung durch Kühlvorrichtungen angewandt wird. Es versteht sich, dass ein vernachlässigbar kleiner Teil der bei der exothermen Reaktion freiwerdenden Wärmemenge unvermeidlich auch vom Reaktorkörper aufgenommen und durch Wärmeleitung und -abstrahlung an die Umwelt abgegeben wird. Im technischen Sinne wird deshalb unter einer adiabatischen Reaktionsführung oder Betriebsweise eine Reaktionsführung oder Betriebsweise verstanden, bei der, abgesehen von dem durch natürliche Wärmeleitung und -abstrahlung vom Reaktor an die Umgebung abgegebenen Teil der Reaktionswärme, die gesamte Reaktionswärme vom Reaktionsgemisch aufgenommen und mit diesem aus dem Reaktor abgeführt wird. Es bestehen zwei grundsätzliche Möglichkeiten zur Steuerung der Reaktionstemperatur. Da die exotherme Reaktion im Fall des vorliegenden Oligomerisierungsverfahrens durch den Kontakt der Olefine mit dem Katalysator zustande kommt und somit nur im Bereich der Katalysatorschüttung Wärme freigesetzt wird, kann die Temperatur im Reaktor durch Einstellen der Konzentration an Olefin im eintretenden Kohlenwasserstoffgemisch gesteuert werden. Diese wird ihrerseits zweckmäßigerweise durch die Rückführung der vom oligomeren Produkt abgetrennten, nicht umgesetzten Olefine und gesättigten Kohlenwasserstoffe zurück in den Oligomerisierungsreaktor geregelt werden. Da der in den Oligomerisierungsreaktor zurückgeführte Strom einen geringeren Gehalt an reaktiven Olefinen und einen höheren Gehalt an unter den Reaktionsbedingungen inerten gesättigten Kohlenwasserstoffe hat als der frisch zugeführte Einsatzkohlenwasserstoffstrom, bewirkt der diesem Strom zugemischte Rückführstrom eine Verdünnung des Olefingehaltes. Über das verhältnis von Rückführstrom zu frischen Einsatzkohlenwasserstoffstrom kann daher mittelbar die Reaktortemperatur gesteuert werden.

Eine weitere Möglichkeit der Verfahrensteuerung besteht in der Regelung der Eintrittstemperatur des Kohlenwasserstoffgemisches. Eine tiefere Temperatur des eintretenden Kohlenwasserstoffgemischs führt zu einer verbesserten Abführung der Reaktionswärme. Andererseits kann beim Nachlassen der Katalysatoraktivität die Eintrittstemperatur des Kohlenwasserstoffgemisches höher gewählt werden, um eine höhere Reaktionsgeschwindigkeit zu erreichen und damit die nachlassende Katalysatoraktivität zu kompensieren. Die Eintrittstemperatur des Kohlenwasserstoffgemischs ist in der Regel durch Sicherheitsaspekte und praktische Erwägungen begrenzt. Die maximale Eintrittstemperatur liegt für ein überwiegend Butene und gegebenenfalls Butane enthaltendes Kohlenwasserstoffgemisch im Allgemeinen bei 130°C. Ist die maximale Eintrittstemperatur des olefinreichen Kohlenwasserstoffgemisches erreicht, ist der Katalysator erschöpft und muß durch neuen Katalysator ersetzt werden. Der erschöpfte Katalysator kann gegebenenfalls regeneriert werden.

Die Betriebsphase des erfindungsgemäßen Verfahrens wird vorzugsweise so gesteuert, dass die Temperaturtönung über ein Katalysatorbett nicht mehr als 50°C, insbesondere nicht mehr als 40°C, besonders bevorzugt nicht mehr als 30°C beträgt. Als Temperaturtönung wird die Differenz zwischen der Eintrittstemperatur des Kohlenwasserstoffgemischs und der Austrittstemperatur des Reaktionsgemischs betrachtet. Die adiabatische Betriebsweise umfasst auch eine Verfahrenskonfiguration des erfindungsgemäßen Verfahrens, bei der die Umsetzung der Olefine zu Oligomeren auf eine Reaktorkaskade aus zwei oder mehreren, vorzugsweise zwei, Oligomerisierungsreaktoren verteilt wird und das teilumgesetzte Reaktionsgemisch nach Verlassen des einen Reaktors und vor Eintritt in den nachfolgenden Reaktor der Kaskade mittels herkömmlicher Kühlvorrichtungen, wie Kühlmäntel oder Wärmetauscher, gekühlt wird.

Bei geeigneter Fahrweise wird pro Katalysatorbett ein Umsatz, bezogen auf den Olefinanteil des Kohlenwasserstoffgemischs, von 15 bis 50 Gew.-% erreicht.

Die Erfindung wird durch die folgenden Beispiele näher veranschaulicht.

### Vergleichsbeispiel

In einen adiabatischen Reaktor (Länge: 4 m, Durchmesser: 80 cm) wurde ein Katalysator eingefüllt, der gemäß DE-4339713 in Form von Tabletten mit den Abmessungen 5 mm x 5 mm hergestellt wurde. (Zusammensetzung in Gew.-% der Aktivkomponenten: NiO 50 Gew.-%, TiO₂ 12,5 Gew.-%, SiO₂ 33,5 Gew.-%, Al₂O₃ 4 Gew.-%.)

Der Katalysator wurde zunächst 24 h bei 160°C mit einem trockenen N₂-Strom konditioniert und danach im Stickstoffstrom auf 25°C abgekühlt. Anschließend wurde der Katalysator bei einem Druck von 4 bar mit einem Kohlenwasserstoffgemisch der folgenden Zusammensetzung und einer Temperatur von 25°C durchspült:

| | |
|---|---|
| i-Butan | 2 Gew.-Teile |
| n-Butan | 10 Gew.-Teile |
| i-Buten | 2 Gew.-Teile |
| 1-Buten | 32 Gew.-Teile |
| trans-2-Buten | 37 Gew.-Teile |
| cis-2-Buten | 17 Gew.-Teile |
| Diolefine + Alkine | < 1 Gew.-ppm |

Dabei kam es innerhalb von 120 min zu einem Temperaturanstieg auf 195°C. Danach wurde die Zufuhr von frischem Kohlenwasserstoffgemisch unterbrochen. Zur Abkühlung des Reaktors wurde der Reaktoraustrag gekühlt, von den gebildeten Oligomeren befreit und der von den Oligomeren befreite Reaktoraustrag zurückgeführt und wieder in den Reaktor eingespeist. Die Rückführung des gekühlten, von den Oligomeren befreiten Austrags wurde fortgesetzt, bis der Reaktor auf 25 °C abgekühlt war. Nach dem Abkühlen auf 25°C wurde erneut ein Gemisch der obigen Zusammensetzung mit einem Durchsatz von 15 kg/h über den Katalysator geleitet. Die Temperatur des eintretenden Gemisches wurde so lange erhöht, bis im Reaktoraustrag ein Buten-Umsatz von 25% festgestellt wurde. Hierzu war eine Eintrittstemperatur von 90°C erforderlich.

Nach Abstellen der Anlage wurde der Katalysator ausgebaut und es wurde eine Zunahme des C-Gehaltes von ursprünglich 3,3 Gew.-% beim frischen Katalysator auf 11,2 Gew.-% bei der Ausbauprobe festgestellt.

### Erfindungsgemäßes Beispiel

Es wurden der im vorstehenden Beispiel beschriebene Reaktor und Katalysator verwendet.

Der Katalysator wurde zunächst 24 h bei 160°C mit einem trockenen N₂-Strom konditioniert und danach im Stickstoffstrom auf 25°C abgekühlt. Anschließend wurde der Katalysator bei einem Druck von 4 bar mit einem Gemisch der folgenden Zusammensetzung und einer Temperatur von 25°C durchspült:

| | |
|---|---|
| i-Butan | 10 Gew.-Teile |
| n-Butan | 50 Gew.-Teile |
| i-Buten | 1 Gew.-Teile |
| 1-Buten | 2 Gew.-Teile |
| trans-2-Buten | 30 Gew.-Teile |
| cis-2-Buten | 7 Gew.-Teile |
| Diolefine + Alkine | < 50 Gew.-ppm |

Dabei kam es innerhalb von 3 h zu einem Temperaturanstieg von 20°C. Der Katalysator wurde weitere 2 h mit dem beschriebenen Gemisch gespült, bis die Temperatur des austretenden Gemisches 25°C betrug. Anschließend wurde ein Gemisch der folgenden Zusammensetzung mit einem Durchsatz von 15 kg/h über den Katalysator geleitet:

| | |
|---|---|
| i-Butan | 2 Gew.-Teile |
| n-Butan | 10 Gew.-Teile |
| i-Buten | 2 Gew.-Teile |
| 1-Buten | 32 Gew.-Teile |
| trans-2-Buten | 37 Gew.-Teile |
| cis-2-Buten | 17 Gew.-Teile |
| Diolefine + Aline | < 1 Gew.-ppm |

Die Temperatur des eintretenden Gemisches wurde so lange erhöht, bis im Reaktoraustrag ein Buten-Umsatz von 25% festgestellt wurde. Hierzu war eine Eintrittstemperatur von 40°C notwendig. Die gegenüber dem vergleichsbeispiel niedrigere Eintrittstemperatur zur Erzielung des gleichen Umsatzes zeigt die höhere Aktivität des erfindungsgemäß vorbehandelten Katalysators. Die anfänglich niedrige Eintrittstemperatur führt zu einer gegenüber dem Vergleichsbeispiel 8-fachen Katalysatorstandzeit.

Nach Abstellen der Anlage wurde der Katalysator ausgebaut und es wurde eine Zunahme des C-Gehaltes von ursprünglich 3,3 Gew.-% beim frischen Katalysator auf 7,9 Gew.-% bei der Ausbauprobe festgestellt.

## Patentansprüche

1. Verfahren zur Oligomerisierung von C₂- bis C₈-Olefinen an einem Nickel enthaltenden heterogenen Katalysator, das umfasst:
i) eine Katalysatorvorbehandlungsphase, bei der der Katalysator mit einem ersten Kohlenwasserstoffgemisch, das einen Olefingehalt von weniger als 50 Gew.-% und einen Gehalt an Diolefinen und Alkinen von weniger als 0,1 Gew.-% aufweist, in Kontakt gebracht wird, und
ii) eine Betriebsphase, bei der ein Strom eines zweiten Kohlenwasserstoffgemisches, das einen Gehalt an C₂- bis C₈-Olefinen von 50 bis 100 Gew.-% und einen Gehalt an Diolefinen und Alkinen von weniger als 10 Gew.-ppm aufweist, mit dem Katalysator in Kontakt gebracht wird.

2. Verfahren nach Anspruch 1, bei dem der Katalysator vor dem Inkontaktbringen mit dem ersten Kohlenwasserstoffgemisch in Gegenwart eines Inertgases auf eine Temperatur von mehr als 100 °C erwärmt wird.

3. Verfahren nach Anspruch 1 oder 2, bei dem die Katalysatorvorbehandlungsphase und die Betriebsphase im gleichen Reaktor durchgeführt werden.

4. Verfahren nach einem der Ansprüche 1 bis 3, bei dem der Katalysator Nickeloxid enthält.

5. Verfahren nach Anspruch 4, bei dem der Katalysator im Wesentlichen aus NiO, SiO₂, TiO₂ und/oder ZrO₂ sowie gegebenenfalls Al₂O₃ besteht.

6. Verfahren nach einem der vorhergehenden Ansprüche, bei dem das erste Kohlenwasserstoffgemisch und der Strom des zweiten Kohlenwasserstoffgemisches vor dem Inkontaktbringen mit dem Katalysator über ein Adsorptionsmaterial geleitet werden.

7. Verfahren nach einem der vorhergehenden Ansprüche, bei dem die Betriebsphase unter einem solchen Druck durchgeführt wird, dass der Strom des zweiten Kohlenwasserstoffgemisches in flüssiger Phase vorliegt.

8. Verfahren nach einem der vorhergehenden Ansprüche, bei dem der Strom des zweiten Kohlenwasserstoffgemisches als wesentliche Bestandteile enthält:
- 50 bis 100 Gew.-% Butene und
- 0 bis 50 Gew.-% Butane.

9. Verfahren nach einem der vorhergehenden Ansprüche, bei dem das erste Kohlenwasserstoffgemisch im Wesentlichen aus C₄-Kohlenwasserstoffen besteht.

10. Verfahren nach einem der vorhergehenden Ansprüche, bei dem die Katalysatorvorbehandlungsphase und die Betriebsphase in einem adiabatischen Reaktor oder einer Kaskade in Reihe geschalteter adiabatischer Reaktoren durchgeführt werden.

## Claims

1. A process for the oligomerization of C₂-C₈-olefins over a nickel-containing heterogeneous catalyst, which comprises:
i) a catalyst pretreatment phase in which the catalyst is brought into contact with a first hydrocarbon mixture having an olefin content of less than 50% by weight and a content of diolefins and alkynes of less than 0.1% by weight, and
ii) an operating phase in which a stream of a second hydrocarbon mixture having a C₂-C₈-olefin content of from 50 to 100% by weight and a content of diolefins and alkynes of less than 10 ppm by weight is brought into contact with the catalyst.

2. A process as claimed in claim 1, wherein the catalyst is heated to a temperature above 100°C in the presence of an inert gas prior to being brought into contact with the first hydrocarbon mixture.

3. A process as claimed in claim 1 or 2, wherein the catalyst pretreatment phase and the operating phase are carried out in the same reactor.

4. A process as claimed in any of claims 1 to 3, wherein the catalyst comprises nickel oxide.

5. A process as claimed in claim 4, wherein the catalyst consists essentially of NiO, SiO₂, TiO₂ and/or ZrO₂ and, if desired, Al₂O₃.

6. A process as claimed in any of the preceding claims, wherein the first hydrocarbon mixture and the stream of the second hydrocarbon mixture are passed over an adsorbent prior to being brought into contact with the catalyst.

7. A process as claimed in any of the preceding claims, wherein the operating phase is carried out at a pressure under which the stream of the second hydrocarbon mixture is liquid.

8. A process as claimed in any of the preceding claims, wherein the stream of the second hydrocarbon mixture comprises as essential constituents:
- from 50 to 100 % by weight of butenes and
- from 0 to 50% by weight of butanes.

9. A process as claimed in any of the preceding claims, wherein the first hydrocarbon mixture consists essentially of C₄-hydrocarbons.

10. A process as claimed in any of the preceding claims, wherein the catalyst pretreatment phase and the operating phase are carried out in an adiabatic reactor or in a cascade of adiabatic reactors connected in series.

## Revendications

1. Procédé d'oligomérisation d'oléfines en C₂ à C₈ sur un catalyseur hétérogène contenant du nickel, qui comprend:
i) une phase de prétraitement du catalyseur, dans laquelle le catalyseur est mis en contact avec un premier mélange d'hydrocarbures, présentant une teneur en oléfines de moins de 50% en poids et une teneur en dioléfines et alkines de moins de 0,1% en poids, et
ii) une phase de travail, dans laquelle un courant d'un deuxième mélange d'hydrocarbures, présentant une teneur en oléfines en C₂ à C₈ de 50 à 100% en poids et une teneur en dioléfines et alkines de moins de 10 ppm en poids, est mis en contact avec le catalyseur.

2. Procédé selon la revendication 1, dans lequel le catalyseur est, avant sa mise en contact avec le premier mélange d'hydrocarbures, chauffé en présence d'un gaz inerte à une température de plus de 100°C.

3. Procédé selon la revendication 1 ou 2, dans lequel la phase de prétraitement du catalyseur et la phase de travail sont entreprises dans le même réacteur.

4. Procédé selon l'une des revendications 1 à 3, dans lequel le catalyseur contient de l'oxyde de nickel.

5. Procédé selon la revendication 4, dans lequel le catalyseur consiste essentiellement en NiO, SiO₂, TiO₂ et/ou ZrO₂ et éventuellement Al₂O₃.

6. Procédé selon l'une des revendications qui précèdent, dans lequel le premier mélange d'hydrocarbures et le courant du deuxième mélange d'hydrocarbures sont, avant leur mise en contact avec le catalyseur, guidés sur un matériau d'adsorption.

7. Procédé selon l'une des revendications qui précèdent, dans lequel la phase de travail est entreprise sous une pression telle que le courant du deuxième mélange d'hydrocarbures se trouve en phase liquide.

8. Procédé selon l'une des revendications qui précèdent, dans lequel le courant du deuxième mélange d'hydrocarbures contient comme principaux constituants:
- de 50 à 100% en poids de butène et
- de 0 à 50% en poids de butane.

9. Procédé selon l'une des revendications qui précèdent, dans lequel le premier mélange d'hydrocarbures est essentiellement constitué d'hydrocarbures en C₄.

10. Procédé selon l'une des revendications qui précèdent, dans lequel la phase de prétraitement du catalyseur et la phase de travail sont entreprises dans un réacteur adiabatique ou dans une cascade de réacteurs adiabatiques reliés en série.
